# EUROPÄISCHE PATENTANMELDUNG

(11) **EP 4 074 263 A1**
(43) Veröffentlichungstag der Anmeldung: **19.10.2022**
(21) Anmeldenummer: 21168365.1
(22) Anmeldetag: 14.04.2021
(51) Int. Cl.: A61B 10/00, A61B 5/15

(54) **KÖRPERASPIRAT ZUR VERWENDUNG IN DER DIAGNOSE**

(71) Anmelder: Görgner, Anke, 04229 Leipzig (DE)
(72) Erfinder: Görgner, Anke, 04229 Leipzig (DE)
(74) Vertreter: Heubeck, Christian

(57) **Zusammenfassung**

Gegenstand der vorliegenden Anmeldung ist eine nicht-invasive Vorrichtung zur Entnahme von Körperaspirat, die Verwendung dieser nicht-invasiven Vorrichtung zur Bereitstellung von Körperaspirat sowie die Verwendung des entsprechend bereitgestellten Körperaspirats zu diagnostischen Zwecken.

## Beschreibung

Gegenstand der vorliegenden Anmeldung ist eine nicht-invasive Vorrichtung zur Entnahme von Körperaspirat, die Verwendung dieser nicht-invasiven Vorrichtung zur Bereitstellung von Körperaspirat sowie die Verwendung des entsprechend bereitgestellten Körperaspirats zu diagnostischen Zwecken.

Verfahren, bei denen auf einem begrenzten Hautareal ein Unterdruck erzeugt wird, sind als traditionelle Therapieverfahren wie beispielsweise Schröpfen seit langem bekannt. Dabei wird mit sogenannten Schröpfgläsern oder Schröpfköpfen ein Unterdruck erzeugt, so dass verstärkt Blut in den geschröpften Bereich fließt. Schröpfen findet als "trockenes Schröpfen" oder "blutiges Schröpfen" Anwendung. Beim blutigen Schröpfen wird vordem Erzeugen von Unterduck auf der Haut die Haut verletzt, wie beispielsweise mit einer Blutlanzette. Der angelegte Unterdruck zieht nachfolgend Blut und insbesondere Kapillarblut durch die Verletzungen heraus.

Das beim blutigen Schröpfen bereitgestellte Kapillarblut ist Blut, welches aus den Haargefäßen (d.h. Kapillargefäßen) des Körpers gewonnen wird. Es findet bei einer Vielzahl von Laboruntersuchungen und besonders in der Schnelldiagnostik mit Teststreifen zur Blutzuckerspiegelbestimmung bei Diabetes mellitus Anwendung. Als "quasiarterielles Blut", das noch keine vollständige Gewebepassage durchlaufen hat, können sich Messwerte für bestimmte Stoffe wie beispielsweise Glukose, Aminosäuren, Proteine etc. von Messwerten für Vollblut unterscheiden.

Das durch blutiges Schröpfen bereitgestellte Kapilliarblut eignet sich deshalb nicht für Bestimmung aller Messwerte, die in Vollblut bestimmt werden können und insbesondere nicht für die Bestimmung von Tumormarkern. Tumormarker sind üblicherweise Substanzen in Blut, die bei Tumorerkrankungen in erhöhter Konzentration auftreten. Sie können vom Körper als Reaktion auf Krebs oder von Krebszellen selbst gebildet werden. Häufig eignen sich diese Marker dazu, den Verlauf aber auch den Erfolg einer Krebstherapie zu beurteilen.

Da Tumormarker üblicherweise in Vollblut bestimmt werden, sind aber nur solche Werte oder Marker in der Analyse auffällig, die systemisch, also im ganzen Körper, zirkulieren. Die gezielte Diagnostik von Änderungen in regional beschränkten Körperbereichen kann durch derartige Vollblutanalysen jedoch nicht detektier oder erfasst werden.

Aufgabe der vorliegenden Erfindung war deshalb die Bereitstellung einer Vorrichtung, welche zum einen die gezielte Diagnostik von regionalen Körperbereichsveränderungen ermöglicht, gleichzeitig aber nicht unter den Limitierungen von Verfahren zur Bereitstellung von Kapillarblut, wie beispielsweise Schröpfen, leidet.

Gelöst wird die Aufgabe der vorliegenden Erfindung durch eine nicht invasive Vorrichtung (100) zur Körperaspiratentnahme, wobei die nicht-invasive Vorrichtung (100) umfasst: eine Saugkammer (110) mit einer behandlungsseitigen Öffnung (120) um Hautgewebe einem in der Saugkammer (110) vorhandenem Behandlungsdruck auszusetzen, und einem Anschluss für eine Unterdruckquelle (130) zur Erzeugung des Behandlungsdrucks, dadurch gekennzeichnet, dass die nicht-invasive Vorrichtung (100) wenigstens einen weiteren Anschluss zur Entnahme von Körperaspirat (140) aufweist.

"Vorrichtung" im Sinne der vorliegenden Erfindung bezieht sich auf die erfindungsgemäße "nicht-invasive Vorrichtung" (100) wie hierin definiert.

"Körperaspirat" im Sinne der vorliegenden Erfindung bezeichnet die körpereigene Substanz, die durch Anwendung der erfindungsgemäßen Vorrichtung bereitgestellt wird. Körperaspirat unterscheidet sich Viskosität, Farbe deutlich von Kapillarblut und ist hiervon scharf abzugrenzen. Während Kapillarblut nur an sehr gut durchbluteten Körperstellen wie Fingerkuppe oder Ohrläppchen entnommen werden kann, kann Körperaspirat prinzipiell an jeder beliebigen Körperstelle entnommen werden. Ferner verschließen sich bei der Kapillarblutentnahme aus dem Kapillarbett während der Entnahme die Blutgefäße so schnell, dass in der Regel nur geringe Mengen von bis zu 0,5 ml Kapillarblut pro Entnahme bereitgestellt werden können. Erfindungsgemäßes Körperaspirat kann hingegen pro Entnahmevorgang bzw. Entnahmestelle mit Volumina von wenigstens 20 ml bereitgestellt werden.

Die erfindungsgemäße, nicht-invasive Vorrichtung (100) kann aus jedem beliebigen Material, welches geeignet ist, dem angelegten Behandlungsdruck stand zu halten, ausgebildet sein. In bevorzugten Ausführungsformen ist die nicht-invasive Vorrichtung aus Kunststoff, wie beispielsweise Polyethylen oder Acrylkunststoff, oder Glas ausgebildet.

Die erfindungsgemäße Vorrichtung kann durchsichtig oder blickdicht gestaltet sein. Bei der Verwendung von durchsichtigem Material kann der Anwender optisch beurteilen, wie sich der angelegte Behandlungsdruck bzw. Unterdruck auf die Körperstelle oder Körperregion auswirkt. Eine blickdichte Gestaltung kann insbesondere für die Körperaspiratentnahme an Stellen, welche für die zu behandelnde Person einsehbar sind, vorteilhaft sein. Erfahrungsgemäß reagiert eine nicht geringe Zahl von Personen beim Anblick von Blut oder Körperaspiratentnahme mit Kreislaufschwäche. In einer bevorzugten Ausführungsform kann die erfindungsgemäße Vorrichtung zum überwiegenden Teil blickdicht gestaltet sein, jedoch kann sie einen durchsichtigen Bereich als Kontrollfenster für den Anwender aufweisen.

Bei "Behandlungsdruck" im Sinne der vorliegenden Anmeldung handelt es sich um einen Unterdruck. Der Behandlungsdruck beispielsweise im Bereich -0,1 bis -1,0 bar zu halten und/oder einzuregeln. In einer bevorzugten Ausführungsform wird als Unterdruckquelle eine Vakuumpumpe und insbesondere eine medizintechnisch zugelassene Vakuumpumpe eingesetzt. Bevorzugt weist diese Pumpe einen stufenlos regelbaren Unterdruckerzeuger auf. In einer anderen Ausführungsform findet eine Handpumpe Anwendung.

Die erfindungsgemäße Vorrichtung weist bevorzugt einen Anschluss für eine Unterdruckquelle (130) auf, welcher mittig und gegenüberliegend zu einer von der behandlungsseitigen Öffnung definierten Grundfläche positioniert ist. In einer bevorzugten Ausführungsform ist der Anschluss für die Unterdruckquelle (130) als Einwegventil ausgebildet. Im Anschluss für die Unterdruckquelle kann das notwendige Ventil fest verbaut sein. In einer anderen Ausführungsform wird in den Anschluss für die Unterdruckquelle vor Verwendung der erfindungsgemäßen Vorrichtung ein Ventil eingebracht. Der Anschluss für die Unterdruckquelle (130) ist bevorzugt konisch, insbesondere von der Saugkammer weggerichtet konisch zulaufend, ausgebildet. Der außerhalb der Saugkammer befindliche Innendurchmesser des Anschlusses für die Unterdruckquelle (130) beträgt dabei bevorzugt ca. 5 mm.

Die Seitenwand S der Saugkammer kann im Bereich des Anschlusses für die Unterdruckquelle (130) verstärkt und/oder verdickt sein. Die Seitenwand S kann im Bereich des Anschlusses (130) einen Einsatz für ein Unterdruckventil und/oder eine Führung aufweisen, deren Ausrichtung senkrecht zu der Fläche ist, die von der behandlungsseitigen Öffnung (120) definiert wird. Die Führung ist vorzugsweise mit der Seitenwand S einstückig und aus demselben Material ausgebildet.

Der Radius der behandlungsseitigen Öffnung (120) kann beispielsweise in Abhängigkeit von der zu behandelten Körperstelle oder Körperregion variieren. Die behandlungsseitige Öffnung weist bevorzugt einen Innendurchmesser (ID) zwischen 50 mm und 90 mm, stärker bevorzugt zwischen 60 mm und 80 mm und am stärksten bevorzugt zwischen 65 mm und 75 mm auf. Ein Innendurchmesser von 72 mm ist insbesondere bevorzugt. Der Außendurchmesser (AD) beträgt bevorzugt wischen 65 und 105 mm, bevorzugt 89 mm. In einer bevorzugten Ausführungsform weist die behandlungsseitige Öffnung (120) eine abgerundete Kante auf. Eine solche Ausgestaltung ist bei der Anwendung für die behandelte Person weniger schmerzhaft. In einer anderen Ausführungsform weist die behandlungsseitige Öffnung (120) einen verbreiterten Randabschluss (121) auf. So wird zum einen ein seitliches Ansaugen von Luft vermieden und zum anderen wiederum der Anwendungskomfort erhöht. Der verbreiterte Randabschluss (121) kann ebenfalls abgerundet sein. Darüber hinaus kann der verbreiterte Randabschluss (121) einen einfacheren Eingriff eines Verschlussmittels, wie beispielsweise eines Dichtgummis oder eines Drehverschluss, für die behandlungsseitige Öffnung (120) ermöglichen. Bevorzugt ist der Randabschluss (121) als Kunststoffgewinde (122) ausgebildet.

Der verbreiterte Randabschluss (121) bzw. das Kunststoffgewinde (122) weist bevorzugt eine Stärke SR zwischen 6 mm und 8 mm, bevorzugt 7 mm und 8 mm, insbesondere ca. 7,5 mm auf. Seine Länge LR beträgt bevorzugt zwischen 11 mm und 14 mm, bevorzugt zwischen 12 mm und 13 mm und insbesondere ca. 12,7 mm.

Die Seitenwand S der Saugkammer (110) kann jede beliebige unter angelegtem Unterdruck stabile Stärke S aufweisen. Das Volumen der Saugkammer (110) kann wiederum in Abhängigkeit von der zu behandelten Körperstelle oder Körperregion variieren. In einer bevorzugten Ausführungsform weist die Saugkammer (110) ein Volumen zwischen 100 cm³ und 300 cm³, stärker bevorzugt zwischen 150 cm³ und 250 cm³ und am stärksten bevorzugt zwischen 200 cm³ und 230 cm³ auf. Ein Volumen von ca. 215 cm³ ist insbesondere bevorzugt. Selbstverständlich ist es möglich, anstelle einer nicht-invasiven Vorrichtung (100) mit großem Volumen mehrere Vorrichtungen mit kleinerem Volumen zu verwenden - falls die zu behandelnde Körperstelle dies zulässt, wie beispielsweise der Rücken.

In einer weiteren bevorzugten Vorrichtung beträgt die Gesamtlänge L (senkrecht gemessen von der durch die behandlungsseitigen Öffnung definierten Grundfläche bis zum äußersten Ende des Anschlusses für die Unterdruckquelle (130)) der Vorrichtung zwischen 60 mm und 90 mm. In einer bevorzugten Ausführungsform beträgt die Gesamtlänge L einschließlich Dichtungsfläche, (z.B. aufschraubarer Deckel) ca. 82 mm.

Die Seitenwand der Saugkammer ist im Bereich der behandlungsseitigen Öffnung (120) vorzugsweise mit Elementen zum Verschluss der behandlungsseitigen Öffnung (120), d.h. einer entfernbaren Dichtungsfläche, ausgebildet. So kann der Verschluss der Öffnung bzw. deren Abdichtung beispielsweise mittels eines passenden Dichtgummis oder mittels eines Deckels mit Drehverschluss, z.B. einem verschraubbaren Deckel (150), erfolgen. In einer bevorzugten Ausführungsform ist der Deckel mit Drehverschluss (150) aus dem gleichen Material wie die erfindungsgemäße Saugkammer ausgebildet. Der Verschluss der Öffnung schließt Körperaspirat in der Saugkammer bevorzugt luftdicht ab und vermeidet oder reduziert so Oxidationsprozesse im Körperaspirat.

Zusätzlich zum Anschluss für eine Unterdruckquelle (130) weist die erfindungsgemäße Vorrichtung wenigstens einen weiteren Anschluss (140) auf, der zur Entnahme von Körperaspirat ausgebildet ist. Bevorzugt weist der Anschluss zur Entnahme des Körperaspirats (140) einen Innendurchmesser von 4 mm bis 5 mm, insbesondere ca. 4,5 mm auf. In einer bevorzugten Ausführungsform ist der Anschluss zur Entnahme des Körperaspirats (140) als Luer-System ausgebildet. In einer bevorzugten Ausführungsform ist der weitere Anschluss zur Entnahme des Körperaspirats (140) zum Anschluss für die Unterdruckquelle (130) auf der Oberfläche der erfindungsgemäßen Vorrichtung bzw. Saugkammer in einem Winkel zwischen 25° und 40°, stärker bevorzugt zwischen 30° und 35° und am stärksten bevorzugt etwa 32,5° angeordnet.

Ein weiterer Aspekt der vorliegenden Erfindung betrifft ein Körperaspirat, welches durch die Verwendung der erfindungsgemäßen nicht-invasiven Vorrichtung bereitgestellt wird.

Das durch das nachfolgend beschriebene Verfahren bereitgestellte Körperaspirat wird regional an der Körperstelle oder Körperregion gewonnen, an welcher die erfindungsgemäße nicht invasive Vorrichtung Anwendung findet. Hierdurch wird es möglich, lokale Veränderungen im Körper zu erfassen bevor eine Erkrankung oder wenigstens diese Erkrankung charakterisierende Parameter auf den Gesamtorganismus übergreifen. Lokal entnommenes Körperaspirat kann so eine deutlich höhere Konzentration an zu detektierenden lokalspezifischen Analyten aufweisen als Vollblut bzw. intervenös entnommenes Blut. So enthält Körperaspirat, welches an einer für Kolonkarzinom spezifischen Körperstelle entnommen wurde beispielsweise (z.B. Brustwirbel Th 10 bis Th 12 links)eine über 100-fach höhere Konzentration an SCC (Squamous cell carinoma antigen)-Marker.

Das erfindungsgemäß bereitgestellte Körperaspirat ermöglicht somit sowohl sensitivere als auch lokal gezielte bzw. organspezifische Diagnostik im Sinne der Früherkennung. Die Diagnostik kann hier sowohl die Veränderung von Blutbestandteilen, Entzündungsmarkern (Cytokinen) zellfreien Nukleinsäuren wie DNA- und/oder RNA-Fragmenten und/oder Tumormarker betreffen. In einer weiteren erfindungsgemäßen Ausführungsform ist auch die Bestimmung zirkulierender Tumorzellen (CTC) oder zirkulierender epithelialer Tumorzellen (CETC) möglich. Es handelt sich dabei um Zellen, welche sich vom Primärtumor gelöst haben und im Blutkreislauf zirkulieren. Diese Blutuntersuchungen sind einfach und sicher durchzuführen und erlauben u.a. den Krankheitsverlauf eines Patienten ohne Analyse solider Tumore mittels invasiver Verfahren zu überwachen und gegebenenfalls Therapien anzupassen.

Die Lage der Körperstellen oder Körperregionen zur Körperaspiratentnahme kann beispielsweise durch Tastbefund und/oder Zuordnung der zu untersuchenden Organe mittels Head-Zonen erreicht werden.

Wie dem Fachmann bekannt, wird Head-Zone als Körperstelle oder Hautbereich definiert, in dem aufgrund des gegliederten Körperaufbaus eine über das zugehörige Rückenmarksegment laufende Querverbindung zwischen somatischem und vegetativem Nervensystem besteht. Der bestimmte Körperstelle oder dem Hautbereich sind dabei bestimmte innere Organe zugeordnet. Die Head'sche Zone, die einem bestimmten Organ zugeordnet ist, kann sich über mehrere Dermatome erstrecken, weist jedoch einen reflektorisch bedeutsamen Maximalpunkt auf. Erfindungsgemäß beschreibt Dermatom dabei den von Rückenmarksnerven (Spinalnerven) sensibel innervierten segmentalen Hautbereich bzw. die entsprechende Körperstelle. In einer bevorzugten Ausführungsform erfolgt die Entnahme von Körperaspirat an wenigstens diesem Maximalpunkt. In einer anderen Ausführungsform wird Körperaspirat an allen dem zu untersuchenden Organ zugeordneten Dermatomen entnommen.

Das Verfahren zur Entnahme von Körperaspirat unter Verwendung der erfindungsgemäßen Vorrichtung umfasst folgende Schritte:
a) Aufsetzen der nicht-invasiven Vorrichtung an der Körperstelle oder Körperregion, an welcher Körperaspirat entnommen werden soll und Anlegen eines Unterdrucks, um Hautgewebe einem in der Saugkammer vorhandenem Behandlungsdruck auszusetzen bzw. anzusaugen. Es ist darauf zu achten, dass die nicht-invasive Vorrichtung (100) vollständigen Kontakt zum Hautgewebe bzw. zur Hautoberfläche hat, um so ein Ansaugen von Luft zu vermeiden.
   Die Dauer des Ansaugschritts (a) beträgt bevorzugt zwischen 30 und 90 Sekunden.
b) Deaktivierung des Vakuummittels und Druckausgleich in der Saugkammer zur Abnahme der nicht-invasiven Vorrichtung.
c) Desinfizieren des zuvor von der behandlungsseitigen Öffnung der nicht-invasiven Vorrichtung umfassten Hautgewebes bzw. der Körperstelle oder Körperregion.
d) Aktivieren des desinfizierten Hautbereichs.
e) Erneutes Aufsetzen der nicht-invasiven Vorrichtung an der gleichen Körperstelle oder Körperregion wie unter Schritt (a) und Anlegen des Behandlungsdrucks mittels Unterdruckquelle über einen definierten Zeitabschnitt. Es ist wiederum darauf zu achten, dass die nicht-invasive Vorrichtung (100) vollständigen Kontakt zum Hautgewebe bzw. zur Hautoberfläche hat, um ein Ansaugen von Luft zu vermeiden.
   Die Dauer des Ansaugschritts (e) beträgt bevorzugt zwischen 3 und 5 Minuten.
f) Deaktivieren der Unterdruckquelle bzw. des Vakuummittels und Druckausgleich der Saugkammer.
(g) Direkte Entnahme des sich in der nicht-invasiven Vorrichtung bzw. der Saugkammer befindlichen Körperaspirats, oder
   Abnahme der nicht-invasiven Vorrichtung enthaltend das entnommene Körperaspirat von der behandelten Körperstelle oder Körperregion und Verschließen der behandlungsseitigen Öffnung. Das luftdicht abgeschlossene Körperaspirat kann dann zu einem späteren Zeitpunkt aus der verschlossenen Saugkammer entnommen werden.
h) Desinfizieren des vorbestimmten Hautbereichs.

Die Aktivierung des Hautareals ermöglicht den Austritt von Körperaspirat bei angelegtem Unterdruck und kann bevorzugt durch Ritzen und/oder Lanzettierung erfolgen. Dabei kann beispielsweise eine sterile Lanzette verwendet werden. Wie oft die Lanzette senkrecht durch die oberflächliche Haut gestoßen wird, ist dabei von der Größe des Hautbereichs abhängig. Soweit sich bei der Aktivierung des Hautareals Blutstropfen zeigen, sollten diese vor Schritt (e) entfernt werden, beispielsweise mit Hilfe eines Mulltupfers.

Das Körperaspirat kann vor Entfernung der Saugkammer vom behandelten Hautgewebebereich durch den Anschluss zur Entnahme von Körperaspirat entnommen werden. In einer anderen Ausführungsform wird die nicht-invasive Vorrichtung enthaltend das entnommene Körperaspirat nach Deaktivierung des Unterdrucks, Druckausgleich und Abnahme von der behandelten Körperstelle direkt verschlossen. Die Entnahme des Körperaspirats durch den Anschluss (140) kann dann zu einem späteren Zeitpunkt erfolgen.

In bevorzugten Ausführungsformen kann vor Entnahme des Körperaspirats ein Antigerinnungsmittel in die Saugkammer eingebracht werden, z. B. durch den Anschluss der Körperaspiratentnahme. In einer anderen Ausführungsform befindet sich Gerinnungsmittel in dem Aufnahmegefäß, in welches das Körperaspirat aus der Saugkammer verbracht wird.

In einem alternativen oder ergänzenden Verfahrensschritt kann Körperaspirat nach Abnahme der Saugkammer vom Hautgewebebereich Körperaspirat durch die behandlungsseitige Öffnung direkt aus der Saugkammer, d. h. nicht aus dem Anschluss für Körperaspiratentnahme, entnommen werden.

Desinfizierungsschritte erfolgen der entsprechend dem Fachmann bekannten Hygienemaßnahmen. Der Bereich der Körperaspiratentnahme wird abschließend sorgfältig desinfiziert und kann mit einer sterilen Wundauflage abgedeckt oder verbunden werden.

Ein weiterer Aspekt der vorliegenden Anmeldung betrifft die Verwendung eines Körperaspirats, welches durch die Verwendung der erfindungsgemäßen nicht-invasiven Vorrichtung bereitgestellt wurde, zu diagnostischen Zwecken.

Das erfindungsgemäß bereitgestellte Körperaspirat ermöglicht prinzipiell die Bestimmung aller Messwerte, die auch im Vollblut bestimmt werden können, z.B. Leberwerte, Nierenwerte etc. Das Körperaspirat kann dabei sowohl an der entsprechenden Headzone und/oder an jeder anderen Stelle der Körperoberfläche entnommen werden.

Das Körperaspirat kann insbesondere zur Bestimmung von Erythrozyten, Leukozyten, Thrombozyten, Blutfetten, Blutsalzen, Blutzuckern, Proteinen, Enzymen, Stoffwechselprodukten, Entzündungsmarkern (Cytokinen), CTCs (zirkulierenden Tumorzellen), Hormongerinnungsfaktoren, zellfreien Nukleinsäuren, wie DNA und/oder RNA-Fragmenten, und/oder Tumormarkern verwendet werden.

In einer bevorzugten Ausführungsform der Erfindung können die Tumormarker SCC, AFP, CA 15-3, CA 19-9, CA 72-4, CA 125, Calcitonin, CEA, hCG, NSE, PSA, und/oder TG bestimmt werden. Die erfindungsgemäße Diagnostik kann sowohl gezielt bei der Früherkennung als auch bei der Kontrolle des Verlaufs von Tumorerkrankungen eingesetzt werden. Die Bestimmung von SCC ist ganz besonders bevorzugt.

Der Tumormarker SCC (Squamous cell carinoma antigen) ist ein immunologischer Parameter, der auf die Existenz eines Plattenepithelcarcinoms im Körper hinweist. Plattenepithel ist weit verbreitet, es befindet sich an den Organen des Magen- DarmTraktes, des Respirationstraktes, auf denen des Urogenitaltraktes und auf der Haut. Ist der SCC in Venenblut (i.v.) erhöht, besteht der Verdacht eines Plattenepithelcarcinoms (Squamous Cell Carcinom).

Das Problem des i.v. SCC besteht darin, dass
1) SCC i.v. nicht organspezifisch ist, wie es z.B. das prostataspezifische Antigen PSA für Prostata ist. Wenn der SCC i.v. erhöht ist, kann die Lokalisation des Primärtumors oder der Sitz der Metastasen überall da sein, wo sich ein Plattenepithel (Squamous cell) befindet. Deshalb gilt der SCC i.v. als unspezifischer Wert und wird gegenwärtig nur zur Verlaufskontrolle genutzt, und
2) ist SCC i.v. erhöht besteht die Wahrscheinlichkeit der Existenz eines Tumors im höheren Stadium 3 oder 4 mehr als 80 %. Im Stadium 1 ist der SCC i.v. nur unter 3% erhöht.

Bestimmt man SCC jedoch in erfindungsgemäß bereitgestellten Körperaspirat ist das anders:
1) läßt sich durch SCC im Körperaspirat eine genaue Lokalisation des Primärtumors oder der Metastasen vornehmen. Respirationsorgane lassen sich so in den oberen Dermatomen C3 bis TH5, Verdauungsorgane in den mittleren Dermatomen Th6 bis L1 und Urogenitalorgane in den unteren lumbosacralen Dermatomen L2 bis S4 gezielt untersuchen, und
2) da SCC im Körperaspirat im Vergleich zum SCC i.v. um ca. das 60fache erhöht ist, eignet sich SCC im Körperaspirat erstmals als Frühtest für Tumore im Stadium 0-1 .

Als Tumormarker kann SCC u.a. bei Kolonkarzinom, Gebärmutterhalskrebs, Analkrebs, Peniskrebs, Speiseröhrenkrebs bestimmten Formen von Lungenkrebs und/oder Tumoren im Hals-Nasen-Ohren-Bereich erhöht sein.

Alpha-Fetoprotein (AFP) ist ein Tumormarker, welcher u.a. bei Keimzelltumoren der Eierstöcke und des Hodens sowie Lebertumoren erhöht sein kann. Der Marker CA 15-3 kann indikativ für Leberzirrhose, Entzündungen des Körpers oder Brustkrebs sein. CA 19-9 ist ein Marker für Tumore des End- und Dickdarms, des Magens, der Leber, der Gallenwege oder der Bauchspeicheldrüse. Das Krebs-Antigen CA 125 kann indikativ für Tumore der Eierstöcke, Ovarialkarzinom, sein. Einen Hinweis auf Schilddrüsentumore können erhöhte Werte für das Eiweiß Calcitonin und/oder TG

(Thyreoglobulin) darstellen. Neuronen-spezifische Enolase (NSE) kann indikativ für bestimmte Lungenkrebsformen sein. Prostata-spezifisches Antigen (PSA) kann bei erhöhten Werten auf Prostatakrebs hinweisen. Das karcinoembryonale Antigen (CEA) kann u.a. bei Verdacht auf Tumore des Magens, der Lunge, der weiblichen Brust, des Magens, der Bauspeicheldrüse, der Schilddrüse, des Dick- oder Enddarms bestimmt werden. Darüber hinaus ist der Marker auch für Leberzirrhose und Entzündungen spezifisch. Humanes Choriogonadotropin (hCG) kann u.a. indikativ für Tumore der Keimzellen der Eierstöcke und des Hodens eingesetzt detektiert werden. Der Marker CA 72-4 kann auf Eierstockkrebs hinweisen oder auch auf eine entzündete Bauchspeicheldrüse. Die genannten Markerspezifitäten sind beispielhaft und nicht abschließend zu verstehen.

In einer besonders bevorzugten Ausführungsform wird Körperaspirat verwendet um den SCC-Wert zu bestimmen, oder falls mehrere Blutwerte und/oder Tumormarker bestimmt werden, wenigstens den SCC-Wert zu bestimmen.

Erfindungsgemäßes Körperaspirat kann insbesondere zur Diagnose des Zustands oder von Erkrankungen der Schilddrüse, der Lunge, des Ösophagus, der Gallenwege, der Leber, des Pankreas, der Harnblase, der Prostata, der Hoden, des Magens, der weiblichen Brust, des Duodenums, des Dünndarms, des Kolons/Rektums, des Ovars, des Zervix, des Uterus, der Niere, der Haut und/oder des Hirns verwendet werden.

### Figuren

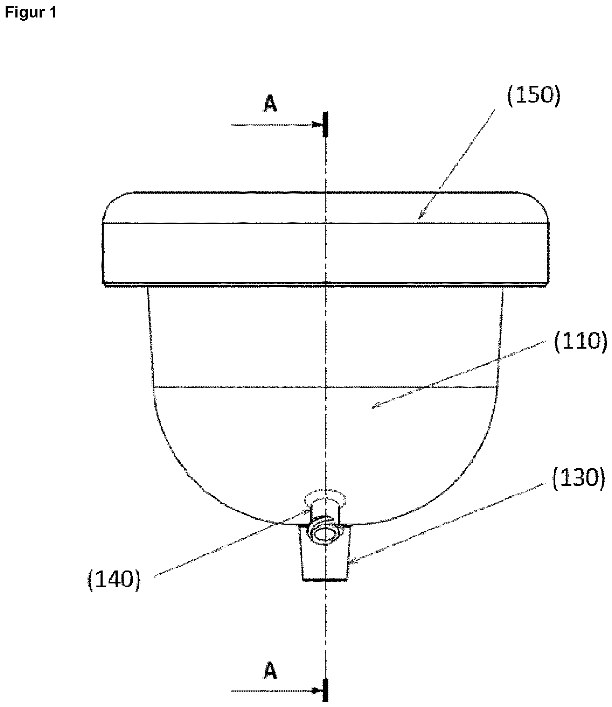

- **Figur 1:**: Nicht-invasive Vorrichtung (100) umfassend eine Saugkammer (100), einen Anschluss für eine Unterdruckquelle (130), einen Anschluss zur Entnahme von Körperaspirat (140) und einen verschaubbaren Deckel (150).

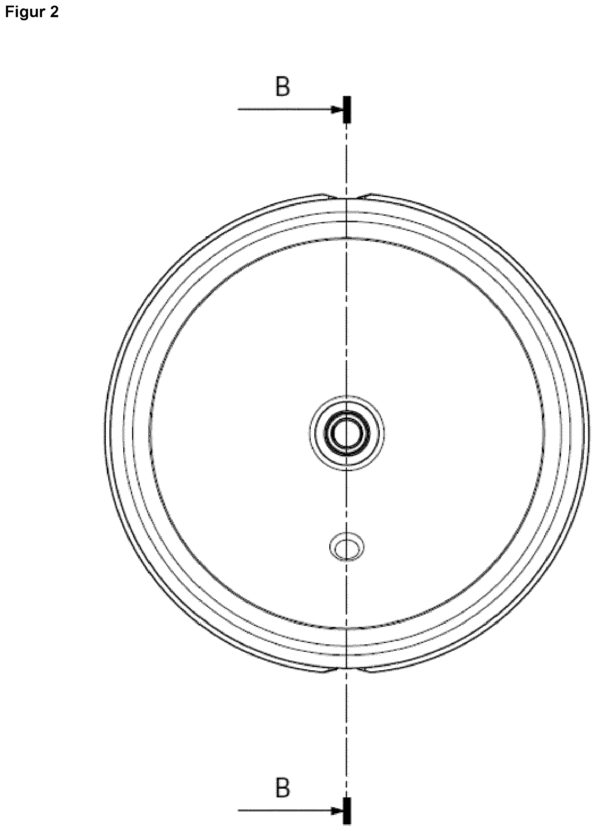

- **Figur 2:**: Aufsicht auf die nicht-invasive Vorrichtung (100).

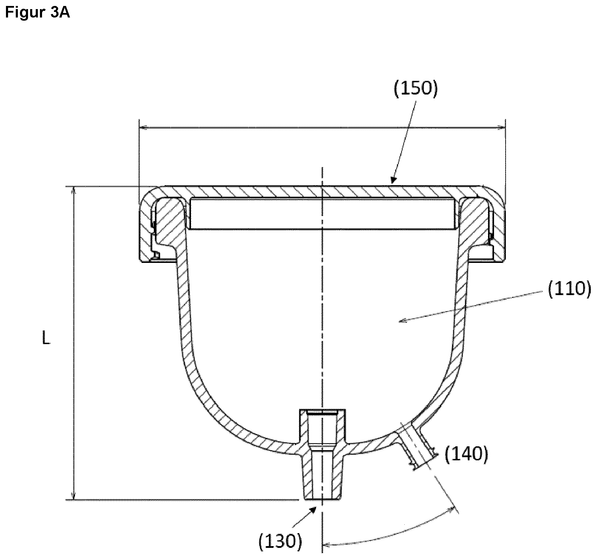

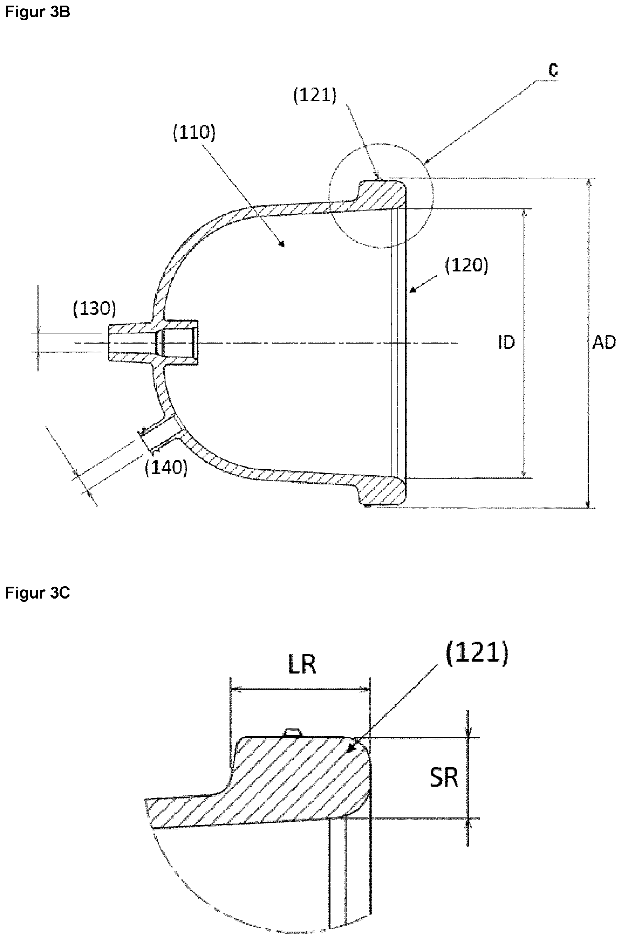

- **Figur 3:**: Querschnittszeichnung der nicht-invasiven Vorrichtung (100) mit verschaubbaren Deckel (150) (**3A**) und ohne verschaubbaren Deckel (**3B**). Angaben sind in mm. Figur **3C** zeigt ein Detail des Kunststoffgewindes (122).

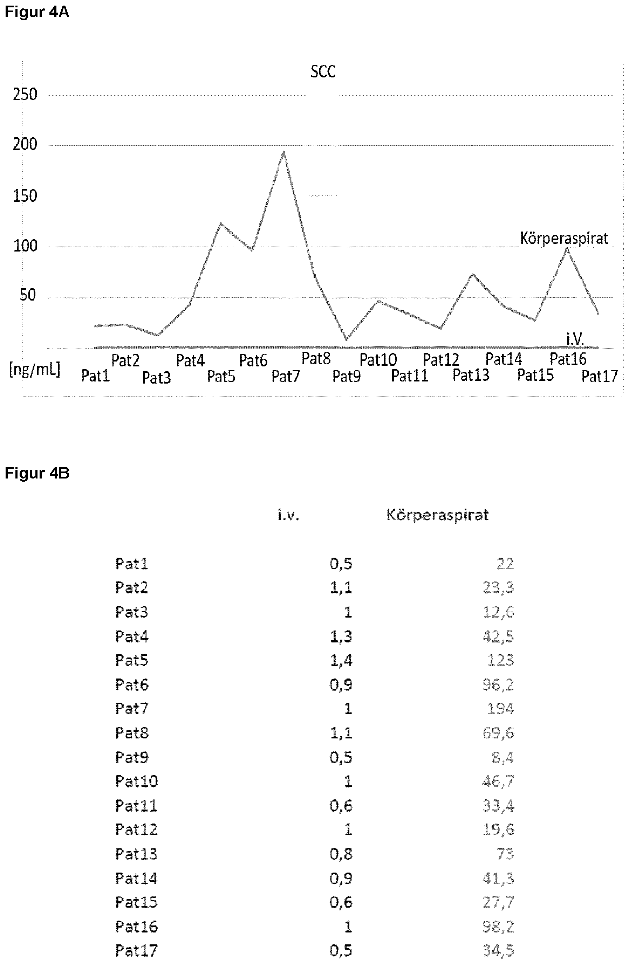

- **Figur 4:**: Figur 4 zeigt Patienten, mit Tumoren am Plattenepithel verschiedener Organe, bei denen der SCC i.v. und SCC im Körperaspirat an den entsprechenden Headzonen entnommen wurde.

### Beispiele

### 1. Kolonkarzinom

Einer 63 jährigen Patientin mit Kolonkarzinom wurde Blut intravenös (Blut i.V.) und Körperaspirat im Sinne der vorliegenden Erfindung entnommen.

| **Tumormarker** | **Blutentnahme i.V.** | **Körperaspirat** | |
|---|---|---|---|
| **CEA** | 1,0 | 1,8 | ng/ml |
| **SCC** | 1,4 | **123,0** | ng/ml |
| **Ca 19-9** | 8,3 | 11,6 | U/ml |

Während die Werte für CEA und Ca 19-9 sowohl für Blut i.V. als auch Körperaspirat unauffällig sind, kann man Körperaspirat ein signifikant höherer Wert (Faktor größer 100) für SCC entsprechend indikativ für Kolonkarzinom detektiert werden. Eine entsprechende Detektion ist mit Blut i.V. nicht möglich; hier bleibt der SCC-Wert unter dem üblichen Grenzwert von 1,5 ng/ml.

### 2. Ovarialkarzinom

Einer 51 jährigen Patientin mit Ovarialkarzinom wurde Blut intravenös (Blut i.V.) und Körperaspirat im Sinne der vorliegenden Erfindung entnommen.

| **Tumormarker** | **Blutentnahme i.V.** | **Körperaspirat** | |
|---|---|---|---|
| **Ca 125** | 38,8 | **55,9** | U/ml |
| **Ca 15-3** | 17,9 | 25,0 | U/ml |
| **TPA** | 26 | 34 | U/l |

Während die Werte für Ca 15-3 und TPA sowohl für Blut i.V. als auch Körperaspirat unauffällig sind, kann man Körperaspirat ein signifikant höherer Wert für Ca 125 entsprechend indikativ für Ovarialkarzinom detektiert werden.

### 3. Prostatakarzinom

Einem 59 jährigem Patienten mit Prostatakarzinom wurde Blut intravenös (Blut i.V.) und Körperaspirat im Sinne der vorliegenden Erfindung entnommen.

| **Tumormarker** | **Blutentnahme i.V.** | **Körperaspirat** | |
|---|---|---|---|
| **Ca 19-9** | 16,4 | 23,1 | U/ml |
| **CEA** | 6,9 | 10,6 | ng/ml |
| **PSA gesamt** | 8,46 | 11,31 | ng/ml |

Alle bestimmten Markerwerte weißen in Körperaspirat ein deutlich stärkeres Überschreiten des Normbereichs auf und geben einen Hinweis auf Prostatakarzinom.

### 4. Rektumkarzinom

Einer 54 jährigen und einer 43 jährigen Patientin mit Rektumkarzinom wurde Blut intravenös (Blut i.V.) und Körperaspirat im Sinne der vorliegenden Erfindung entnommen.

| **Tumormarker** | **Blutentnahme i.V. (54 j./43 j.)** | **Körperaspirat (54 j.)** | **Körperaspirat (43 j.)** | |
|---|---|---|---|---|
| **CEA** | 2,8/85,5 | **11,3** | **94,0** | ng/ml |
| **SCC** | --/1,0 | -- | **22,1** | ng/ml |
| **Ca 19-9** | 11,7/12,0 | 12,3 | 13,3 | U/ml |

Während die Werte für Ca 19-9 in beiden Fällen unauffällig ist, weisen beide Patientinnen in Körperaspirat sowohl für CEA als auch SCC im Vergleich zu Blutentnahme i.V. deutlich erhöhte Werte auf, welche für Rektumkarzinom indikativ sind.

## Patentansprüche

1. Nicht-invasive Vorrichtung (100) zur Entnahme von Körperaspirat,
wobei die nicht-invasive Vorrichtung (100) umfasst: eine Saugkammer (110) mit einer behandlungsseitigen Öffnung (120) um Hautgewebe einem in der Saugkammer (110) vorhandenem Behandlungsdruck auszusetzen, und einem Anschluss für eine Unterdruckquelle (130) zur Erzeugung des Behandlungsdrucks,
**dadurch gekennzeichnet,**
**dass** die nicht-invasive Vorrichtung (100) wenigstens einen weiteren Anschluss zur Entnahme von Körperaspirat (140) aufweist.

2. Nicht-invasive Vorrichtung (100) nach Anspruch 1,
**dadurch gekennzeichnet,**
**dass** die Vorrichtung (100) aus Kunststoff oder Glas ausgebildet ist.

3. Nicht-invasive Vorrichtung (100) nach einem der Ansprüche 1 oder 2,
**dadurch gekennzeichnet,**
**dass** der Anschluss für die Unterdruckquelle (130) gegenüberliegend und mittig zu einer von der behandlungsseitigen Öffnung (120) definierten Grundfläche positioniert ist.

4. Nicht-invasive Vorrichtung (100) nach einem der Ansprüche 1 bis 3,
**dadurch gekennzeichnet,**
**dass** die Saugkammer (110) ein Volumen zwischen 100 cm³ und 300 cm³, stärker bevorzugt zwischen 150 cm³ und 250 cm³, und am stärksten bevorzugt zwischen 200 cm³ und 230 cm³ aufweist.

5. Nicht-invasive Vorrichtung (100) nach einem der Ansprüche 1 bis 4,
**dadurch gekennzeichnet,**
**dass** behandlungsseitige Öffnung (120) einen Innendurchmesser ID zwischen 60 mm und 80 mm aufweist.

6. Nicht-invasive Vorrichtung (100) nach einem der Ansprüche 1 bis 5,
**dadurch gekennzeichnet,**
**dass** der Anschluss für die Unterdruckquelle (130) als Einwegventil ausgebildet ist.

7. Nicht-invasive Vorrichtung (100) nach einem der Ansprüche 1 bis 6,
**dadurch gekennzeichnet,**
**dass** die Seitenwand der Saugkammer (110) im Bereich der behandlungsseitigen Öffnung (120) mit Elementen zum Verschluss der behandlungsseitigen Öffnung ausgebildet ist.

8. Nicht-invasive Vorrichtung (100) nach einem der Ansprüche 1 bis 7,
**dadurch gekennzeichnet,**
**dass** der Verschluss ein zweiteiliger Verschluss ist, wobei der Verschluss bevorzugt mittels eines Dichtgummis (160) oder als Drehverschluss, insbesondere als verschraubbarer Deckel (150) erfolgt.

9. Nicht-invasive Vorrichtung (100) nach einem der Ansprüche 1 bis 8,
**dadurch gekennzeichnet,**
**dass** die Seitenwand S der Saugkammer (110) im Bereich der behandlungsseitigen Öffnung (120) einen verbreiterten Randabschluss (121), insbesondere ein Kunststoffgewinde (122), aufweist, wobei der verbreiterte Randabschluss (121) bevorzugt eine Stärke SR zwischen 6 mm und 8 mm und/oder eine Länge LR zwischen 11 mm und 14 mm aufweist.

10. Nicht-invasive Vorrichtung (100) nach einem der Ansprüche 1 bis 9,
**dadurch gekennzeichnet,**
**dass** der Anschluss zur Entnahme von Körperaspirat (140) zum Anschluss für die Unterdruckquelle in einem Winkel von 25° bis 40°, stärker bevorzugt 30° und 35°, angeordnet ist.

11. Nicht-invasive Vorrichtung (100) nach einem der Ansprüche 1 bis 10,
**dadurch gekennzeichnet,**
**dass** der Anschluss zur Entnahme von Körperaspirat (140) als Luer-System ausgebildet ist.

12. Körperaspirat bereitgestellt durch die Verwendung einer nicht-invasiven Vorrichtung nach einem der Ansprüche 1 bis 11.

13. Verwendung des Körperaspirats nach Anspruch 12 zu diagnostischen Zwecken.

14. Verwendung eines Körperaspirats des Anspruch 13 zur Bestimmung von Erythrozyten, Leukozyten, Thrombozyten, Blutfetten, Blutsalzen, Blutzuckern, Entzündungsmarkern (Cytokinen), zirkulierenden Tumorzellen (CTCs), Proteinen, Enzymen, Stoffwechselprodukten, Hormonen, Gerinnungsfaktoren und/oder Tumormarkern, insbesondere SCC, CA 15-3, CA 19-9, CA 125, Calcitonin, TG, PSA, NSE, CEA, hCG, CA 72-4 TPA und/oder CEA..

15. Verwendung des Körperaspirats nach Anspruch 13 oder 14 zur Diagnose des Zustands oder von Erkrankungen der Schilddrüse, der Lunge, des Ösophagus, der Gallenwege, der Leber, des Pankreas, der Harnblase, der Prostata, der Hoden, des Magens, der weiblichen Brust, des Duodenums, des Dünndarms, des Kolons/Rektums, des Ovars, des Zervix, des Uterus, der Niere, der Haut und des Hirns.
